# Europäisches Patentamt

## European Patent Office

## Office européen des brevets

(19)

(11) Numéro de publication: **0 170 550**
**B1**

(12) **FASCICULE DE BREVET EUROPEEN**

(45) Date de publication du fascicule du brevet:
**16.08.89**

(51) Int. Cl.⁴: **A 61 K 37/16,** A 61 K 37/18,
C 07 K 1/12, C 07 K 5/08,
C 07 K 7/06

(21) Numéro de dépôt: **85401208.5**

(22) Date de dépôt: **18.06.85**

(54) **Nouvelles substances biologiquement actives obtenues a partir de la caseine bovine, leur procédé de preparation et les compositions qui les contiennent.**

(30) Priorité: **19.06.84 FR 8409561**

(43) Date de publication de la demande:
**05.02.86 Bulletin 86/6**

(45) Mention de la délivrance du brevet:
**16.08.89 Bulletin 89/33**

(84) Etats contractants désignés:
**AT BE CH DE FR GB IT LI LU NL SE**

(56) Documents cités:
**EP-A- 0 033 686**
**FR-A- 2 491 334**
**FR-A- 2 513 881**
**US-A- 3 558 770**

(73) Titulaire: **RHONE-POULENC SANTE, 20, avenue Raymond Aron, F-92160 Antony (FR)**

(72) Inventeur: **Jolles, Pierre, 2, rue Jean-François GERBILLON, F-75270 Paris Cedex 06 (FR)**
Inventeur: **Migliore-Samour, Danièle, 64/66, avenue Charles Gide, F-94270 Le Kremlin-Bicetre (FR)**
Inventeur: **Parker, Fabienne, 32, avenue Paul Painlevé, F-942000 St Maur-Des-Fosses (FR)**

(74) Mandataire: **Pilard, Jacques et al, RHONE-POULENC RECHERCHES Service Brevets Pharma 25, Quai Paul Doumer, F-92408 Courbevoie Cedex (FR)**

## Description

La présente invention concerne des substances biologiquement actives obtenues par fractionnement d'hydrolysats enzymatiques de la caséine bovine et les compositions qui les contiennent.

Les substances selon la présente invention sont des agents immunologiques qui favorisent en particulier la production d'anticorps.

Selon la présente invention, les nouvelles substances sont obtenues par traitement, à l'aide d'au moins une enzyme protéolytique, de la caséine bovine totalement délipidée suivi du fractionnement des produits hydrosolubles en fonction de leur poids moléculaire moyen par filtration et purification sur des supports appropriés.

Les nouvelles substances selon l'invention ont un poids moléculaire moyen compris entre 300 et 2500 et présentent des propriétés particulièrement intéressantes.

A titre d'enzymes protéolytiques, on utilise avantageusement dans le procédé selon l'invention la trypsine, la chymotrypsine ou autre enzyme analogue ou leurs mélanges.

Par exemple, le fractionnement de la fraction hydrosoluble provenant du traitement de la caséine bovine délipidée par un mélange chloroforme-méthanol, digérée par la trypsine non prétraitée fournit trois fractions biologiquement actives IV, V et VI.

L'ensemble des fractions «IV + V», après purification sur une colonne d'échangeur d'ions, puis par chromatographie liquide à haute performance dans des conditions déterminées, fournit les substances appelées «MJH 320». «MJH 324» «MJH 328» et «MJH 329» dont les propriétés immunologiques sont améliorées par rapport à celles des substances IV et V elles-mêmes.

La purification ultérieure de la substance «MJH 320» permet d'isoler à titre d'exemple le pentapeptide Phe-Phe-Ser-Asp-Lys qui correspond au peptide 17–21 de la para kapa-caséine bovine [J. Jollès et coll., Chimia 26 (12) 645–646 (1972)].

La purification ultérieure de la substance «MJH 328» ou le fractionnement plus étalé de la fraction donnant naissance à la substance «MJH 328» permet de séparer par exemple les fractions «MJH 362» et «MJH 365» à partir desquelles sont respectivement isolés et caractérisés le tripeptide Leu-Leu-Tyr qui correspond au peptide 191–193 de la caséine β-bovine [R. Ribadeau-Dumas et coll., Eur. J. Biochem., Eur., 25, 505–514 (1972)] et l'hexapeptide Thr-Thr-Met-Pro-Leu-Trp qui correspond au peptide C-terminal de la caséine αS₁ bovine [J.C. Mercier et coll., Eur. J. Biochem., 23, 41–51 (1971)].

Les nouvelles substances selon la présente invention sont des agents immunologiques qui favorisent la production d'anticorps et qui accélèrent le phénomène de phagocytose.

In vitro, elles se sont montrées particulièrement actives à des concentrations comprises entre 0,1 et 10 µg/ml dans le test de sécrétion d'anticorps (hémolytiques) antiglobules rouges de moutons par des cellules spléniques de souris immunisées in vivo et dans le test de phagocytose de globules rouges de moutons opsonisés par les macrophages péritonéaux de souris.

Les exemples suivants, donnés à titre non limitatif, montrent comment l'invention peut être mise en pratique.

Exemple 1

La caséine bovine (500 mg) est délipidée par traitement au moyen de 25 cm³ d'un mélange chloroforme-méthanol (⅔ en volumes).

La caséine est solubilisée par traitement au moyen d'une solution d'hydroxyde de sodium 0,05 M de telle manière que la concentration finale en caséine soit voisine de 2 mg/cm³.

La solution obtenue est soumise à une dialyse contre 2 litres de tampon phosphate 0,033 M à pH 8 pendant 2 jours en renouvelant 5 fois la solution tampon.

On obtient ainsi une solution à pH 8 contenant la caséine soluble. Cette solution est soumise à l'action de la trypsine de telle manière que le rapport enzyme/substrat soit voisin de ¹/₁₀₀. L'hydrolyse enzymatique est poursuivie pendant 24 heures à 37 °C, la moitié de la quantité d'enzyme étant introduite au départ de la réaction et le reste 4 heures après de début de la digestion.

Le mélange réactionnel est amené à sec, puis repris par 16 cm³ d'une solution d'acide acétique à 30%. Le mélange est soumis à une centrifugation à 15 000 tours/minute, pendant 45 minutes.

Le liquide clair surnageant (16 cm³) est filtré sur une colonne de Sephadex G 50 (hauteur: 117 cm. diamètre 4,5 cm) en éluant avec de l'acide acétique à 30% et en recueillant des fractions de 3 cm³.

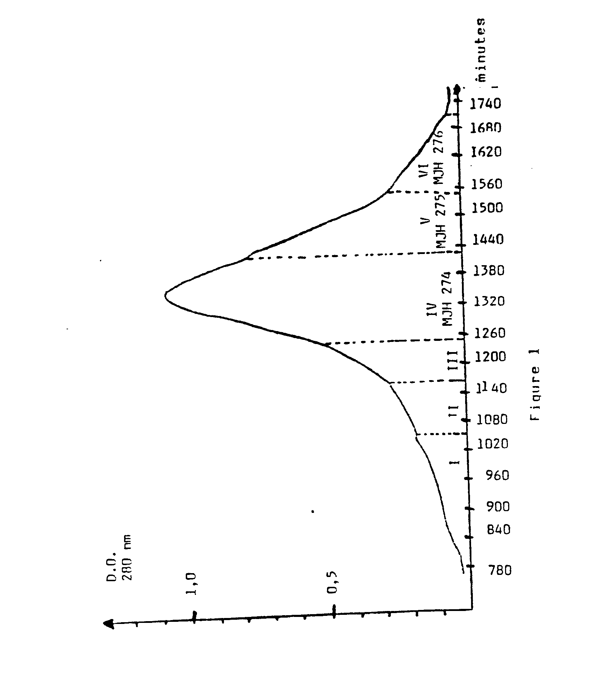

En opérant de cette manière et en suivant la chromatographie par absorption U.V. à 280 nm, on obtient des zones qui permettent de définir 6 fractions pour lesquelles le poids moléculaire moyen est établi. Le diagramme de la filtration est représenté à la figure 1.

Les fractions biologiquement actives sont les suivantes:
– substance IV (MJH 274): fractions 1248 cm³ à 1419 cm³; poids moléculaire moyen 1000 ± 250
– substance V (MJH 275): fractions 1420 cm³ à 1554 cm³; poids moléculaire moyen 600 ± 250
– substance VI (MJH 276): fractions 1555 à 1695 cm³; poids moléculaire moyen 400 ± 100

Les substances IV et V réunies (1,5 g provenant de 3 opérations successives) sont filtrées sur une colonne de CM Trisacryl (nom déposé I B F) (hauteur 110 cm, diamètre 2,2 cm) en éluant par une solution de tampon Tris, HCl [Tris (hydroxyméthylaminométhane) HCl] 0,01 M à pH 4,5 (1090 cm³), puis par une solution tampon Tris, HCl 0,01 M pH 4,5, ClNa 0,1 M (1040 cm³), les fractions recueillies étant de 3,35 cm³.

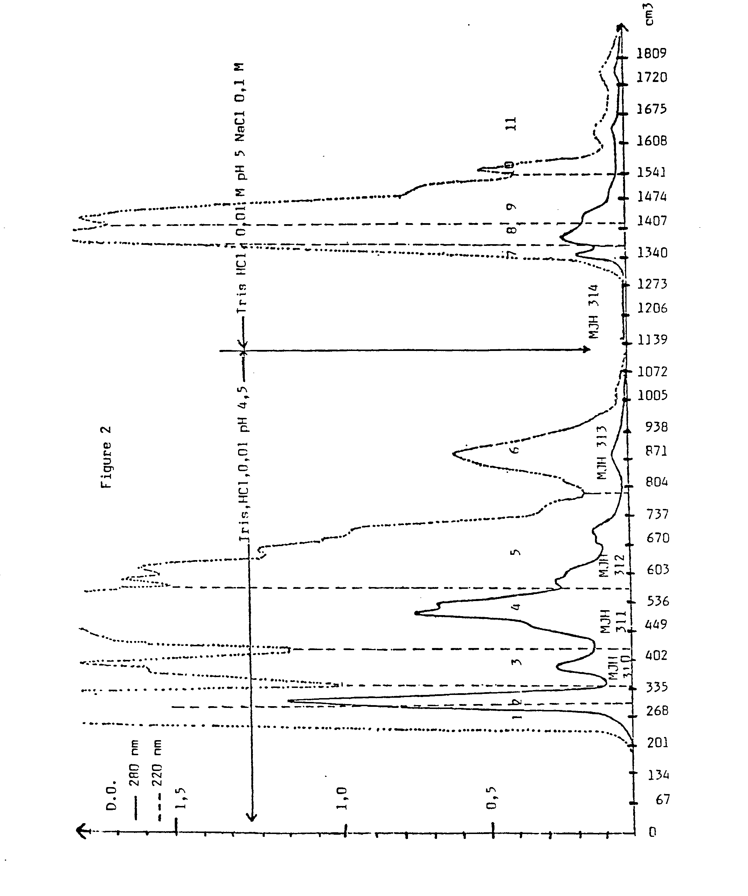

Le diagramme de filtration est représenté par la figure 2 dans laquelle figurent en abscisses le volume d'élution et en ordonnées les densités optiques. On recueille 11 fractions parmi lesquelles les fractions éluées de 335 cm³ à 435 cm³ (MJH 310). de 435 cm³ à 570 cm³ (MJH 311), de 570 cm³ à 787 cm³ (MJH 312), de 787 cm³ à 1010 cm³ (MJH 313) et de 1306 cm³ à 1374 cm³ (MJH 314) se sont révélées actives.

Les fractions MJM 311 et MJH 313 ont été purifiées successivement par HPLC en phase reverse sur une colonne semi-préparative (colonne C18-μ-bondapak, Waters) dont la longueur est de 30 cm et le diamètre de 7,8 mm. On recueille des fractions de 1 cm³, la vitesse d'élution étant de 1 cm³/minute. Au départ, la colonne est tamponnée par de l'acide trifluoroacétique (TFA) à 0,1% (éluant A). On prépare un éluant contenant du TFA (0,1% en volume) et de l'acétonitrile (70%) (éluant B). La fraction MJH 311 est dissoute dans 1 cm³ de TFA 0,1%.

L'élution est effectuée en utilisant un gradient linéaire d'élution et en opérant selon le tableau suivant:

Temps
| (minutes) | Eluant A | Eluant B |
|---|---|---|
| 25 | 100 | 0 |
| 115 | 0 | 100 |

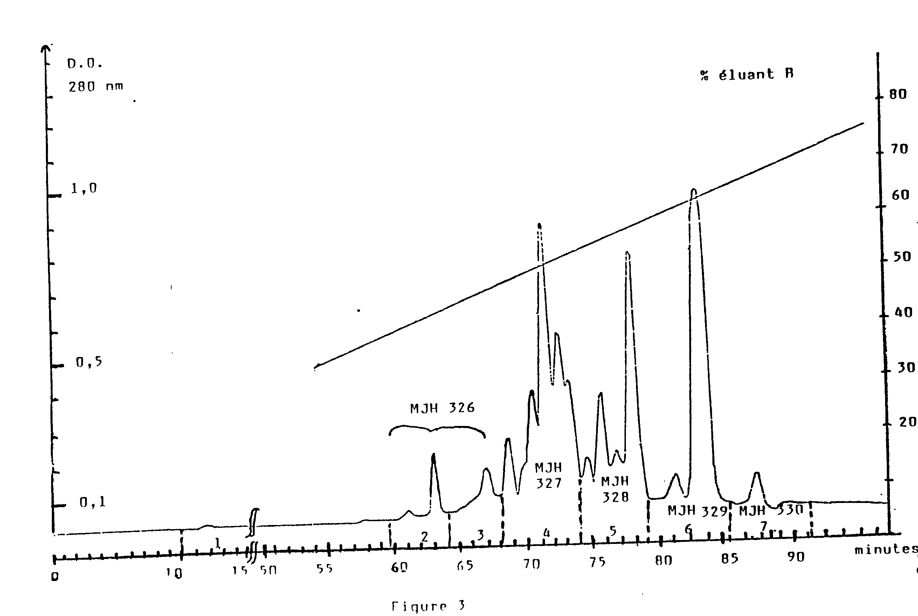

Le diagramme d'élution est représenté par la figure 3. L'élution est suivie en mesurant l'absorption à 280 nm.

Le diagramme d'élution contient 7 zones dont les fractions les plus importantes sont les fractions éluées de 74 à 79 mn (MJH 328) et de 79 à 86 mn (MJH 329).

La fraction MJH 313 est dissoute dans 1 cm³ de TFA 0,1%. L'élution est effectuée en utilisant le gradient d'élution décrit dans le tableau suivant:

Temps
| (minutes) | Eluant A | Eluant B | Gradient |
|---|---|---|---|
| 25 | 100 | 0 | isocratique |
| 55 | 65 | 35 | linéaire |
| 115 | 65 | 35 | isocratique |
| 145 | 0 | 100 | linéaire |

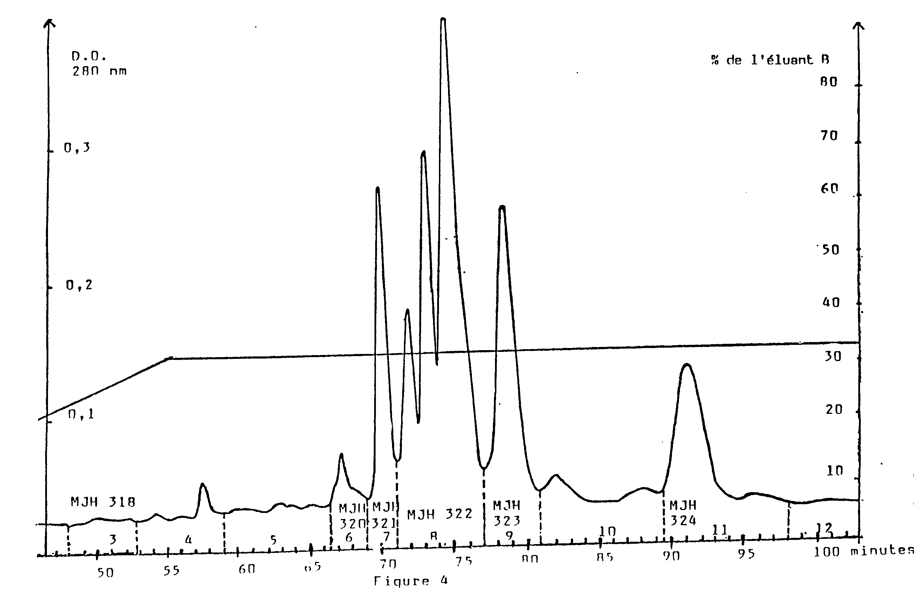

Le diagramme d'élution est représenté par la figure 4. L'élution est suivie en mesurant l'absorption à 280 nm.

Le diagramme d'élution contient 12 zones. Des substances actives ont été mises en évidence dans les zones éluées de 67 à 69 mn (MJH 320) et de 89 à 98 mn (MJH 324).

Exemple 2

La substance «MJH 320» est purifiée par HPLC en phase reverse sur une colonne semi-préparative (colonne C 18-μ-bondapak, Waters) dont la longueur est de 30 cm et le diamètre de 7,8 mm. On recueille des fractions de 0,5 cm³, la vitesse d'élution étant de 1 cm³/minute. Au départ, la colonne est tamponnée par de l'acide trifluoroacétique (TFA) à 0,1% (éluant A). On prépare un éluant contenant du TFA (0,1% en volume) et de l'acétonitrile (70%) (éluant B). La fraction MJH 320 est dissoute dans 1 cm³ de TFA 0,1%.

L'élution est effectuée en utilisant un gradient par paliers isocratiques d'élution et en opérant selon le tableau suivant:

Temps
| (minutes) | Eluant A | Eluant B |
|---|---|---|
| 0 | 100 | 0 |
| 20 | 85 | 15 |
| 60 | 83 | 17 |
| 100 | 78 | 22 |

L'élution est suivie en mesurant l'absorption à 220 nm.

Les fractions éluées entre 29 et 42 minutes sont réunies et purifiées à nouveau dans les même conditions en utilisant le gradient d'élution décrit dans le tableau suivant:

Temps
| (minutes) | Eluant A | Eluant B | Gradient |
|---|---|---|---|
| 0 | 100 | 0 | |
| 20 | 84 | 16 | linéaire |
| 80 | 84 | 16 | isocratique |
| 110 | 83 | 17 | isocratique |

L'élution est suivie en mesurant l'absorption à 220 nm.

Les fractions éluées de 34 à 43 cm³ sont réunies et contiennent le pentapeptide Phe-Phe-Ser-Asp-Lys.

La structure du pentapeptide est déterminée:
– par hydrolyse totale par l'acide chlorhydrique 6N à 110 °C pendant 18 heures qui montre la présence de phénylalanine (Phe) = 2, sérine (Ser) = 1, acide aspartique (Asp) = 1 et lysine (Lys) = 1
– par dansylation, afin de déterminer la nature de l'aminoacide N-terminal, qui est la phénylalanine
– par digestion enzymatique par la carboxypeptidase B, afin de déterminer la nature de l'aminoacide C-terminal, qui est la lysine
– par analyse par le séquenceur Beckmann, Modèle 890 C, programme Quadrol 0,1 M avec détermination des différentes étapes grâce à la caractérisation des phénylthiohydantoïne-acides aminés (détermination par HPLC et révélation sur plaques).

Exemple 3

La fraction «MJH 311» obtenue à l'exemple 1 est purifiée par HPLC en phase reverse sur une colonne semi-préparative (colonne C 18-μ-bondapak, Waters) dont la longueur est de 30 cm et le diamètre de 7,8 mm. On recueille des fractions de 1 cm³, la vitesse d'élution étant de 1 cm³/minute. Au départ, la colonne est tamponnée par de l'acide trifluoroacétique (TFA) à 0,1% (éluant A). On prépare un éluant contenant du TFA (0,1% en volume) et de l'acétonitrile (70%) (éluant B). La fraction «MJH 311» est dissoute dans 1 cm³ de TFA 0,1%.

L'élution est effectuée en utilisant un gradient d'élution et en opérant selon le tableau suivant:

Temps
| (minutes) | Eluant A | Eluant B | Gradient |
|---|---|---|---|
| 15 | 100 | 0 | isocratique |
| 40 | 70 | 30 | linéaire |
| 75 | 65 | 35 | linéaire |
| 105 | 65 | 35 | isocratique |

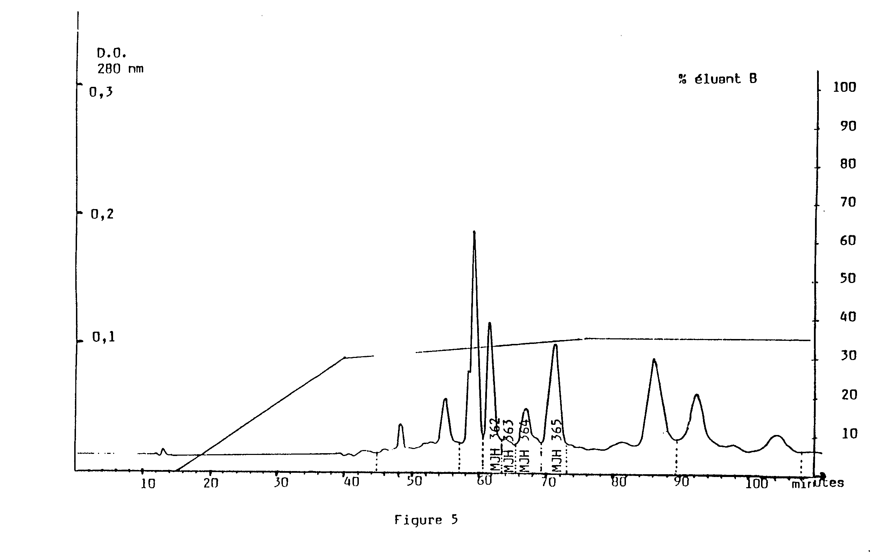

Le diagramme d'élution est représenté par la figure 5. L'élution est suivie en mesurant l'absorption à 280 nm.

Le diagramme d'élution contient 4 zones dont les fractions les plus importantes sont les fractions éluées de 61 à 63 minutes (MJH 362) et de 69 à 73 minutes (MJH 365). La fraction «MJH 365» correspond au pic principal de la fraction «MJH 328» décrit sur la figure 3.

La fraction «MJH 365» est purifiée par HPLC en utilisant une colonne semi-préparative décrite ci-dessus avec les mêmes éluants au même débit et en recueillant des fractions de 0,5 cm³. La fraction «MJH 365» est dissote dans 1 cm³ de TFA 0,1%.

L'élution est effectuée en utilisant un gradient d'élution et en opérant selon le tableau suivant:

| Temps (minutes) | Eluant A | Eluant B | Gradient |
|---|---|---|---|
| 0 | 100 | 0 | |
| 30 | 75 | 25 | linéaire |
| 50 | 75 | 25 | isocratique |
| 110 | 73 | 27 | isocratique |

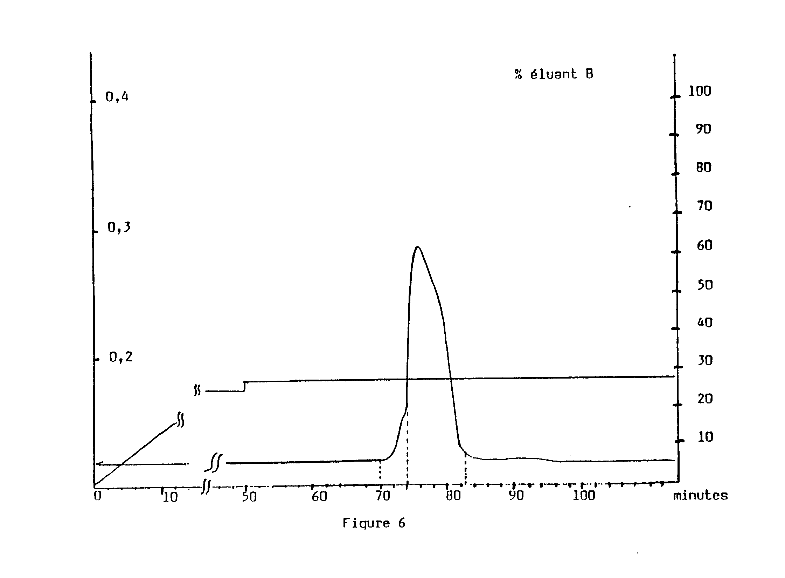

Le diagramme d'élution est représenté par la figure 6. L'élution est suivie en mesurant l'absorption à 280 nm.

Les fractions éluées entre 74,5 et 79 minutes sont réunies et contiennent l'hexapeptide Thr-Thr-Met-Pro-Leu-Trp.

La structure de l'hexapeptide est déterminée:
– par hydrolyse totale par l'acide chlorhydrique 6N à 110 °C pendant 18 heures qui montre la présence de thréonine (Thr) = 2. méthionine (Met) = 1, proline (Pro) = 1. leucine (Leu) = 1.
– par hydrolyse avec l'acide méthanesulfonique 4N contenant 0,2% de tryptamine à 100 °C pendant 18 heures sous pression réduite qui montre la présence de tryptophane (Trp = 1)
– par dansylation afin de déterminer la nature de l'aminoacide N-terminal qui est la thréonine
– par digestion enzymatique par la carboxypeptidase A afin de déterminer la nature de l'aminoacide C-terminal qui est le tryptophane.
– par analyse par le séquenceur Beckmann, Modèle 890 C, programme Quadrol 0,1 M.

Exemple 4

La fraction «MJH 362» séparée à l'exemple 3 est purifiée par HPLC dans les conditions décrites précédemment en recueillant des fractions de 0.5 cm³.

L'élution est effectuée en utilisant un gradient isocratique et en opérant selon le tableau suivant:

| Temps (minutes) | Eluant A | Eluant B |
|---|---|---|
| 0 | 100 | 0 |
| 15 | 100 | 0 |
| 75 | 82 | 18 |

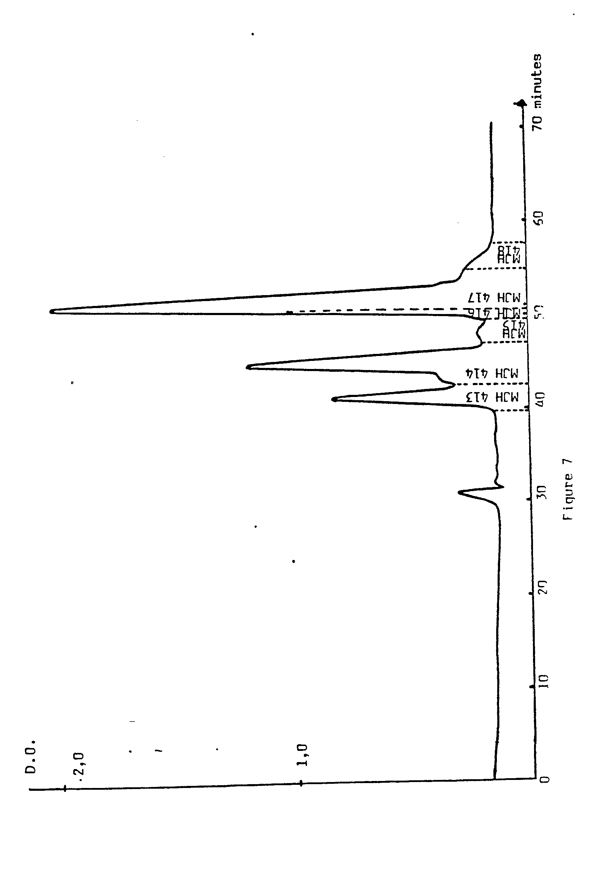

Le diagramme d'élution est représenté par la figure 7. L'élution est suivie en mesurant l'absorption à 280 nm.

Le diagramme d'élution contient 6 zones dont les plus importantes sont éluées de 38,5 à 43 cm³ (MJH 413). de 43.5 à 47 cm³ (MJH 414) et de 52 à 53,5 cm³ (MJH 417).

La fraction MJH 413 contient le tripeptide Leu-Leu Tyr.

La structure du tripeptide est déterminée:
– par hydrolyse totale par l'acide chlorhydrique 6N à 110 °C pendant 18 heures qui montre la présence de leucine (Leu) = 2 et de tyrosine (Tyr = 1)
– par dansylation qui permet de déterminer la nature de l'acide aminé N-terminal qui est la leucine
– par action de la carboxypeptidase A afin de déterminer la nature de l'aminoacide C-terminal qui est la tyrosine.

La présente invention concerne également les compositions utilisables en thérapeutique qui contiennent une substance, selon la présente invention en association avec un ou plusieurs diluants ou adjuvants compatibles et pharmaceutiquement acceptables.

Ces compositions peuvent être utilisées comme adjuvants de vaccins (par exemple vaccin antigrippal composé de sous-unités hémagglutinantes, vaccin antipoliomyélitique à virus inactivé, vaccin antimalarique) en injection simultanée avec l'antigène (viral. bactérien, parasitaire, fongique, tumoral) à l'égard duquel on souhaite augmenter la production d'anticorps ou la réactivité cellulaire spécifique.

Ces compositions pharmaceutiques peuvent être également employées comme immunostimulants non spécifiques en vue d'augmenter la résistance de l'hôte (homme ou animal domestique) vis-à-vis des infections ou en immunothérapie antitumorale.

En tant qu'adjuvants, les produits peuvent être administrés soit en solution aqueuse, soit en émulsion huileuse, soit encore sous forme de liposomes avec l'antigène à l'égard duquel on souhaite obtenir une réponse immunitaire augmentée ou améliorée par la voie utilisée pour cet antigène et dans des proportions variant entre 0,01 et 10 fois la quantité d'antigène avec lequel ils sont mélangés avant d'être injectés.

Pour l'application comme immunostimulant non spécifique, ces produits peuvent être utilisés par voie intraveineuse, intramusculaire, sous-cutanée. intranasale, éventuellement orale ou rectale, soit en solution aqueuse soit en émulsion huileuse, soit encore sous forme de liposomes. Dans ce cas, la dose de composés selon l'invention qui est administrée est généralement comprise entre 0,01 et 10 mg/kg. En thérapeutique humaine, les doses journalières dépendent de l'effet recherché. Elles peuvent être comprises entre 0,5 et 10 mg pour un adulte.

Les compositions solides pour administration orale peuvent être des comprimés, des pilules. des poudres ou des granulés.

Comme compositions liquides pour administration orale, on peut utiliser des émulsions pharmaceutiquement acceptables, des solutions, des suspensions, des sirops et des élixirs.

Les compositions pour administration parentérale ou intranasale peuvent être des solutions stériles aqueuses, des suspensions ou des émulsions.

La stérilisation peut se faire de plusieurs façons. par exemple à l'aide d'un filtre bactériologique ou en incorporant des agents stérilisants. Les compositions solides rendues stériles par irradiation (rayons β) peuvent être dissoutes dans de l'eau stérile ou tout autre milieu stérile injectable éventuellement au moment de l'emploi.

Les compositions pour administration rectale sont des suppositoires.

L'exemple suivant illustre une composition selon l'invention.

### Exemple

On prépare selon la technique habituelle une composition liquide administrable par voie intraveineuse ayant la composition suivante:
- substance MJH-320        10 mg
- soluté injectable        5 cm³

### Revendications

pour les Etats contractants BE, CH, DE, FR, GB, IT, LI, LU, NL, SE

1. Procédé de préparation des nouvelles substances biologiquement actives Phe-Phe-Ser-Asp-Lys, Thr-Thr-Met-Pro-Leu-Tyr et Leu-Leu-Tyr caractérisé en ce que l'on traite la caséine bovine délipidée au moyen d'un mélange chloroforme-méthanol par une enzyme protéolytique choisie parmi la trypsine, la chymotrypsine ou leurs mélanges, le rapport enzyme/substrat étant voisin de 1/100 pendant 24 heures à 37 °C puis, après centrifugation, fractionne les produits hydrosolubles par filtration sur Sephadex G 50 en éluant avec de l'acide acétique à 30%, puis isole les substances actives Phe-Phe-Ser-Asp-Lys, Thr-Thr-Met-Pro-Leu-Tyr et Leu-Leu-Tyr après purification par chromatographie sur échangeurs d'ions et par chromatographie liquide à haute performance.

2. Le pentapeptide Phe-Phe-Ser-Asp-Lys

3. L'hexapeptide Thr-Thr-Met-Pro-Leu-Tyr

4. Le tripeptide Leu-Leu-Tyr

5. Composition pharmaceutique caractérisée en ce qu'elle contient au moins une substance active selon l'une des revendications 2, 3 ou 4 en association avec un ou plusieurs diluants ou adjuvants compatibles.

### Revendication

pour l'Etat contractant AT

Procédé de préparation des nouvelles substances biologiquement actives Phe-Phe-Ser-Asp-Lys, Thr-Thr-Met-Pro-Leu-Tyr et Leu-Leu-Tyr caractérisé en ce que l'on traite la caséine bovine délipidée au moyen d'un mélange chloroforme-méthanol par une enzyme protéolytique choisie parmi la trypsine, la chymotrypsine ou leurs mélanges, le rapport enzyme/substrat étant voisin de 1/100 pendant 24 heures à 37 °C puis, après centrifugation, fractionne les produits hydrosolubles par filtration sur Sephadex G 50 en éluant avec de l'acide acétique à 30%, puis isole les substances actives Phe-Phe-Ser-Asp-Lys, Thr-Thr-Met-Pro-Leu-Tyr et Leu-Leu-Tyr après purification par chromatographie sur échangeurs d'ions et par chromatographie liquide à haute performance.

### Patentansprüche

für die Vertragsstaaten BE, CH, DE, FR, GB, IT, LI, LU, NL, SE

1. Verfahren zur Herstellung von neuen biologisch aktiven Substanzen Phe-Phe-Ser-Asp-Lys, Thr-Thr-Met-Pro-Leu-Tyr und Leu-Leu-Tyr, dadurch gekennzeichnet, daß man entlipidiertes Rinderkasein mit einem Gemisch Chloroform/Methanol durch ein proteolytisches Enzym, ausgewählt unter Trypsin, Chymotrypsin oder deren Gemischen, während 24 Stunden bei 37 °C behandelt, wobei das Verhältnis Enzym/Substrat nahe bei 1/100 liegt, dann nach Zentrifugieren die wasserlöslichen Produkte durch Filtrieren über Sephadex G 50 fraktioniert, wobei mit 30prozentiger Essigsäure eluiert wird, dann die aktiven Substanzen Phe-Phe-Ser-Asp-Lys, Thr-Thr-Met-Pro-Leu-Tyr und Leu-Leu-Tyr nach Reinigung durch Ionenaustauschchromatographie und durch Hochleistungsflüssigkeitschromatographie isoliert.

2. Das Pentapeptid Phe-Phe-Ser-Asp-Lys.

3. Das Hexapeptid Thr-Thr-Met-Pro-Leu-Tyr.

4. Das Tripeptid Leu-Leu-Tyr.

5. Pharmazeutische Zusammensetzung, dadurch gekennzeichnet, daß sie mindestens eine aktive Substanz gemäß einem der Ansprüche 2, 3 oder 4 zusammen mit einem oder mehreren verträglichen Verdünnungs- oder Hilfsmitteln enthält.

### Patentanspruch

für den Vertragsstaat AT:

Verfahren zur Herstellung neuer, biologisch aktiver Substanzen Phe-Phe-Ser-Asp-Lys, Thr-Thr-Met-Pro-Leu-Tyr und Leu-Leu-Tyr, dadurch gekennzeichnet, daß man Rinderkasein, das mit Hilfe einer Mischung Chloroform/Methanol von Lipiden befreit wurde, mit einem proteolytischen Enzym, ausgewählt aus Trypsin, Chymotrypsin oder ihren Mischungen, 24 Stunden bei 37 °C behandelt, wobei das Verhältnis Enzym/Substrat etwa 1/100 ist, und daß man dann nach Zentrifugieren die wasserlöslichen Produkte durch Filtrieren auf Sephadex G 50 fraktioniert, indem man mit 30prozentiger Essigsäure eluiert, und dann die aktiven Substanzen Phe-Phe-Ser-Asp-Lys, Thr-Thr-Met-Pro-Leu-Tyr und Leu-Leu-Tyr nach Reinigung durch Chromatographie auf Ionenaustauschern und durch Hochleistungsflüssigkeitschromatographie isoliert.

### Claims

for the contracting states BE, CH, DE, FR, GB, IT, LI, LU, NL, SE

1. A process for preparing new biologically active substances Phe-Phe-Ser-Asp-Lys, Thr-Thr-Met-Pro-Leu-Tyr and Leu-Leu-Tyr, wherein bovine casein delipidized by means of a chloroform/methanol mixture is treated with a proteolytic enzyme chosen from trypsin, chymotrypsin or mixtures thereof for 24 hours at 37 °C, the enzyme/substrate ratio being in the region of 1:100, then, after centrifugation, the water-soluble products are fractionated by filtration on Sephadex G-50, eluting with 30% strength acetic acid, and the active substances Phe-Phe-Ser-Asp-Lys, Thr-Thr-Met-Pro-Leu-Tyr and Leu-Leu-Tyr are then isolated after purification by ion exchange chromatography and by high performance liquid chromatography.

2. The pentapeptide Phe-Phe-Ser-Asp-Lys.

3. The hexapeptide Thr-Thr-Met-Pro-Leu-Tyr.

4. The tripeptide Leu-Leu-Tyr.

5. A pharmaceutical composition which contains at least one active substance according to one of claims 2, 3 or 4 in combination with one or more diluents or adjuvants which are compatible.

## Claim

for the contracting states AT:

A process for preparing new biologically active substances Phe-Phe-Ser-Asp-Lys, Thr-Thr-Met-Pro-Leu-Tyr and Leu-Leu-Tyr, wherein bovine casein delipidized by means of a chloroform/methanol mixture is treated with a proteolytic enzyme chosen from trypsin, chymotrypsin or mixtures thereof for 24 hours at 37 °C, the enzyme/substrate ratio being in the region of 1:100, then, after centrifugation, the water-soluble products are fractionated by filtration on Sephadex G-50, eluting with 30% strength acetic acid, and the active substances Phe-Phe-Ser-Asp-Lys, Thr-Thr-Met-Pro-Leu-Tyr and Leu-Leu-Tyr are then isolated after purification by ion exchange chromatography and by high performance liquid chromatography.

Figure 1

Figure 2

Figure 3

Figure 4

Figure 5

Figure 6

Figure 7